# EUROPÄISCHE PATENTSCHRIFT

(11) **EP 0 935 946 B1**
(45) Veröffentlichungstag und Bekanntmachung des Hinweises auf die Patenterteilung: **27.11.2002**
(21) Anmeldenummer: 98124727.3
(22) Anmeldetag: 28.12.1998
(51) Int. Cl.: A61B 5/00, A61B 5/024, A61B 5/0404

(54) **Sendereinheit mit elastischen Bändern zur Erfassung des menschlichen Pulses**
Transmitting unit provided with elastic straps for the detection of the human pulse
Emetteur pourvu de rubans flexibles destiné à la détection du pouls humain

(30) Priorität: 16.02.1998 DE 29802596 U
(43) Veröffentlichungstag der Anmeldung: 18.08.1999
(73) Patentinhaber: Schäfer, Jörg, 61273 Wehrheim (DE)
(72) Erfinder: Schäfer, Jörg, 61273 Wehrheim (DE)
(74) Vertreter: Schubert, Siegmar, Dipl.-Ing.

(56) Entgegenhaltungen:
- US-A- 2 096 564
- US-A- 4 806 411
- US-A- 5 178 163
- US-A- 5 464 021

## Beschreibung

Die Erfindung betrifft einen Sender zur Erfassung des menschlichen Pulses mit elastischen Bändern, die flügelartig von dem Sender abstehen, auf einer Innenseite Kontaktflächen einer Sendereinheit aufweisen und insbesondere mittels eines Spannbands um einen Brustkorb spannbar sind, im einzelnen nach dem Oberbegriff des Anspruchs 1.

Sender mit elastischen Bändern zur Erfassung des menschlichen Pulses gehören zum Stand der Technik (z.B. Gebrauchsanweisung Phase 4 der Firma JS Electronic Sports).

Zur zuverlässigen und exakten Messung des Pulses eines Menschen ist es wichtig, den Sender, welcher die eigentliche Sendereinheit und mit dieser in Verbindung stehende elastische Bänder aufweist, die flügelartig von einer Sendereinheit abstehen, auf einer Innenseite Kontaktflächen der Sendereinheit aufweisen und insbesondere mittels eines Spannbands um einen Brustkorb spannbar sind, so auszubilden, daß ein guter elektrischer Kontakt zwischen den Kontaktflächen auf der Innenseite der elastischen Bänder und der Haut der untersuchten Person, beispielsweise unter dem Brustmuskel, herrscht. Gleichwohl soll die untersuchte Person trotz der angelegten elastischen Bänder keine erhebliche Einengung spüren, andererseits aber die Sendereinheit auch beim Bewegen nicht verlieren. Die Kontaktgabe kann durch zusätzliche Mittel, sogenanntes EKG-Gel, verbessert werden. Hierzu ist es jedoch erforderlich, die Kontaktflächen vor jeder Benutzung mit dem EKG-Gel zu bestreichen.

Bei einem bekannten Sender der eingangs genannten Gattung liegen die elastischen Bänder in der Nähe des Senders über streifenförmigen Elektroden, wobei Löcher auf der Oberseite der elastischen Bänder in Fluidkanäle übergehen, die durch die elastischen Bänder und die streifenförmigen Elektroden hindurchreichen (US-A-5 464 021). Die lichten Querschnitte der Löcher erweitern sich von einer Übergangsstelle zu den Fluidkanälen nach außen. Die Fluidkanäle münden in Löcher in den streifenförmigen Elektroden, wobei sich die lichten Querschnitte der Löcher ausgehend von der Innenseite der Elektroden bis auf den Fluidkanalquerschnitt reduzieren. Die Löcher auf der Oberseite der elastischen Bänder dienen dazu, ein leitfähiges Fluid aufzunehmen, welches so durch die Fluidkanäle und die Löcher der Elektroden fließt und die Haut des Trägers unter den Elektroden benetzt. Dabei wird offensichtlich mit großen Querschnitten der Löcher auf den Innenseiten der Elektroden eine großflächige Benetzung angestrebt.

Zum Stand der Technik gehört ferner ein dreilagiger Stützgürtel mit einer Schaumschicht zwischen zwei geschlossenen Faserschichten (US-A-5 178 163). Die Schaumschicht ist mit einer Vielzahl von Durchgangslöchern durchbrochen, die sich von innen nach außen erweitern, damit Schweiß durch nach außen unten gerichtete Flächen der Durchgangslöcher von der inneren Oberfläche abfließen kann, statt sich auf dieser anzusammeln. - Die Wirkung der Durchgangslöcher ist jedoch wegen der geschlossenen inneren und äußeren Faserschichten begrenzt. Ein elektrisches Kontaktproblem tritt nicht auf, die innere Faserschicht ist normalerweise isolierend.

Der vorliegenden Erfindung liegt die Aufgabe zugrunde, einen Sender mit elastischen Bändern mit den weiteren Merkmalen gemäß dem Oberbegriff des Anspruchs 1 so auszubilden, daß eine zuverlässige Kontaktgabe an den Kontaktflächen auf der Unterseite bzw. Innenseite der elastischen Bänder ermöglicht wird, wobei der Sender trotzdem angenehm zu tragen sein soll. Der Sender mit den elastischen Bändern soll zum angenehmen Tragen möglichst leicht sein.

Diese Aufgabe wird erfindungsgemäß durch die Gestaltung der Löcher gemäß dem kennzeichnenden Teil des Anspruchs 1 gelöst.

Durch diese Ausbildung der Löcher werden die elastischen Bänder, die insbesondere aus Gummi bestehen können, massearm und leicht, trotzdem aber mit einer verhältnismäßig großen Kontaktfläche auf der Innenseite der Bänder, ausgebildet, jedenfalls im Vergleich zu Löchern, wie sie sonst bei gelochten Uhrenarmbändern, auch an Herzfrequenzarmbanduhren bekannt sind, die über die Dicke der Bänder konstante lichte Querschnitte aufweisen.

Der Sender mit den erfindungsgemäß mit Löchern versehenen elastischen Bändern hat gegenüber einem solchen mit ungelochten Bändern außerdem den Vorteil, daß die Haut durch die Löcher hindurch transpirieren kann, wodurch der Tragekomfort weiter erhöht wird.

Die elastischen Bänder können besonders massearm, jedoch mit verhältnismäßig guter Kontaktgabe an der Innenseite gemäß Anspruch 2 ausgebildet werden, wonach sich die freien Querschnitte der Löcher von der Innenseite der Bänder im entspannten, ungedehnten Zustand nach außen annährend exponentiell erweitern.

Dadurch sind also die Löcher außen verhältnismäßig groß, während sie auf der Innenseite nur sehr klein sind und die Kontaktfläche nur unwesentlich verringern. Durch den exponentiellen Verlauf der Querschnitte wird außerdem erreicht, daß die Löcher auf der Innenseite der Bänder glatt sind, was für eine gute Kontaktgabe wichtig ist. Auf der Außenseite der Bänder sind diese hingegen weitgehend abgerundet, wodurch die Trageeigenschaften nach außen hin zusätzlich verbessert werden.

Diese vorteilhafte Abrundung ist gemäß Anspruch 4 jedoch auch bei anderen, nicht exponentiellen Erweiterungen der freien Querschnitte der Löcher möglich.

Eine einfachere Form der Erweiterung der freien Querschnitte der Löcher gemäß Anspruch 3 sieht vor, daß sich die Löcher in den Bändern im entspannten, ungedehnten Zustand nach außen annährend konusförmig erweitern.

Vorteilhaft sind die Löcher gemäß Anspruch 5 in Richtung des elastischen Bands im entspannten, ungedehnten Zustand länglich geformt. Bei dieser Formgebung sind die Zugübertragungseigenschaften des Bands trotz verhältnismäßig großer Löcher günstig.

Ebenfalls günstig zur Kraftübertragung und guten Kontaktgabe sind die Löcher gemäß Anspruch 6 über der Innenseite des elastischen Bands annähernd gleichmäßig verteilt.

Ein Ausführungsbeispiel der Erfindung wird im folgenden anhand einer Zeichnung mit zwei Figuren erläutert, die wichtige Merkmale der Erfindung zeigt:
- Fig. 1: einen Teilabschnitt des Senders mit einem auf einer Seite einer Sendereinheit abstehenden elastischen Band im entspannten, ungedehnten Zustand, wobei auf der einen abgebrochenen Seite der dort nicht dargestellten Sendereinheit ein ähnliches elastisches Band absteht, und
- Fig. 2: einen Querschnitt durch das elastische Band gemäß Figur 1 in größerer Darstellung.

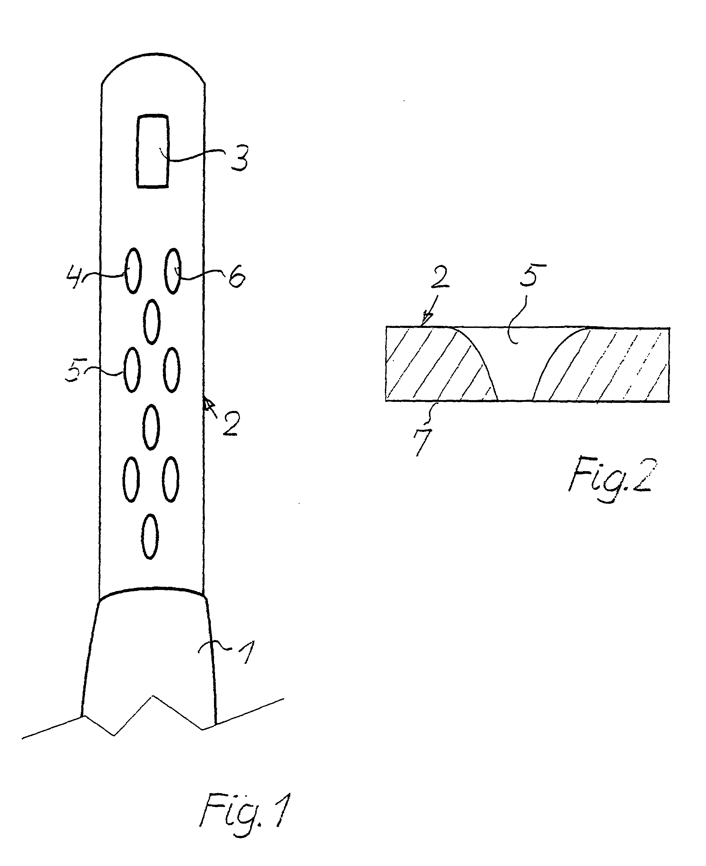

In Fig. 1 ist ein Teil einer Sendereinheit zur Herzfrequenzmessung mit 1 bezeichnet. Die Sendereinheit enthält in an sich bekannter Weise eine Elektronik mit einer austauschbaren Batterie sowie einer Spule, welche in der Elektronik erzeugte Signale, welche die Herzfrequenz beinhalten, zu einer nicht dargestellten Anzeigeeinrichtung überträgt. Die Anzeigeeinrichtung in der äußeren Form einer Armbanduhr empfängt diese Signale, wertet diese aus und zeigt sie auf einem Display an.

Mit der Sendereinheit in mechanischer und elektrischer Verbindung steht auf einander entgegengesetzten Seiten je ein flügelartig von der Sendereinheit 1 abstehendes elastisches Band, von denen nur ein elastisches Band 2 in Fig. 1 dargestellt ist. Das Band besteht aus Gummi, es können in anderen Ausführungsformen statt dessen andere Materialien vorgesehen sein.

Das elastische Band 2 weist an einem Ende eine konventionell ausgeformte Befestigungsöffnung 3 auf. Zwischen der Befestigungsöffnung 3 und der Sendereinheit 1 sind Löcher ausgeformt, die im Querschnitt des elastischen Bands 2 in Fig. 2 vergrößert dargestellt sind und von denen drei Löcher mit 4, 5, 6 bezeichnet sind. Diese Löcher 4,5,6 gehören zu jeweils einer spaltenweise angeordneten Gruppe von Löchern, wobei die vertikalen Abstände der Löcher untereinander in einer Spalte gleich sind. In horizontaler Richtung sind die spaltenweise angeordneten Löcher ebenfalls gleichmäßig versetzt. Die Löcher 4 bis 6 und die weiteren diesen zugeordneten Löcher sind in Hauptrichtung des elastischen Bands 2 länglich, annähernd elliptisch geformt.

Die Vergrößerung der Querschnitte dieser Löcher, z.B. 5, ergibt sich aus Fig. 2, die einen rechtwinklig zu obigen Querschnitten orientierten Querschnitt durch das elastische Band 2 in der Mitte des Lochs 5 darstellt. Eine Innenseite des elastischen Bands 7, welche in Gebrauchslage mit der Person in Kontakt steht, deren Herzfrequenz bzw. Pulsfrequenz gemessen werden soll, ist in Fig. 2 mit 7 bezeichnet.

Wie aus Fig. 2 im einzelnen ersichtlich ist, erweitert sich das Loch 5 von der Innenseite 7 nach außen annähernd exponentiell in der Weise, daß das Loch auf der Innenseite 7 klein und scharf begrenzt ist, während es außen relativ groß ist und abgerundet in die nicht bezeichnete Außenseite übergeht.

Mit einem Sender, der solche elastischen Bänder aufweist, kann die Herzfrequenzmessung bzw. Messung des Pulses an der Brust einer Person sehr genau durchgeführt werden, wobei die Person durch die mittels eines nicht dargestellten Spannbands aufgespannten elastischen Bänder wenig belastet wird.

## Patentansprüche

1. Sender zur Erfassung des menschlichen Pulses mit elastischen Bändern (2), die flügelartig von einer Sendereinheit (1) abstehen, auf einer Innenseite (7) elektrische Kontaktflächen der Sendereinheit, die in Gebranchslage mit einer Person Kontaktierbar sind, aufweisen und insbesondere mittels eines Spannbands um einen Brustkorb spannbar sind, wobei die elastischen Bänder (2) Löcher (4-6) aufweisen, deren freie Querschnitte sich in einem entspannten, ungedehnten Zustand der elastischen Bänder ausgehend von den Kontaktflächen (7) zu einer Außenseite der elastischen Bänder fortlaufend erweitern.

2. Sender nach Anspruch 1,
**dadurch gekennzeichnet,**
**daß** sich die freien Querschnitte der Löcher (4-6) von der Innenseite (7) der Bänder (2) im entspannten, ungedehnten Zustand nach außen annähernd exponentiell erweitern.

3. Sender nach Anspruch 1,
**dadurch gekennzeichnet,**
**daß** sich die freien Querschnitte der Löcher in den Bändern (2) im entspannten, ungedehnten Zustand nach außen annährend konusförmig erweitern.

4. Sender nach einem der Ansprüche 1 bis 3,
**dadurch gekennzeichnet,**
**daß** die Löcher (4-6) auf der Außenseite der Bänder im entspannten, ungedehnten Zustand abgerundet sind.

5. Sender nach einem der Ansprüche 1, 2 und 4,
**dadurch gekennzeichnet,**
**daß** die Löcher (4-6) in Richtung des elastischen Bands (2) im entspannten,ungedehnten Zustand länglich geformt sind.

6. Sender nach einem der vorangehenden Ansprüche,
**dadurch gekennzeichnet,**
**daß** die Löcher (4,6) über der Innenseite (7) des elastischen Bands (2) annähernd gleichmäßig verteilt sind.

## Claims

1. Transmitter for the detection of the human pulse including elastic straps (2) projecting like wings from a transmitting unit (1), on one inner side (7) electric contact faces of said transmitting unit which when used may be brought into contact with a person and which, in particular, may be strapped by means of a tensioning strap about a chest, wherein said elastic straps (2) include holes (4 - 6) the free cross section of which in a relaxed, non-stretched state of the elastic straps continuously enlarges starting from contact faces (7) to one outer side of said elastic straps.

2. Transmitter according to claim 1,
**characterized in**
**that** in the relaxed, non-stretched state said free cross sections of holes (4 - 6) enlarge approximately exponentially from the inner side (7) of said straps (2) to the outside.

3. Transmitter according to claim 1,
**characterized in**
**that** in the relaxed, non-stretched state said free cross sections of said holes in said straps (2) enlarge approximately conically to the outside.

4. Transmitter according to one of claims 1 to 3,
**characterized in**
**that** in the relaxed, non-stretched state, said holes (4 - 6) are rounded on the outer side of said straps.

5. Transmitter according to one of claims 1, 2 and 4,
**characterized in**
**that** in the relaxed, non-stretched state said holes (4 - 6) are longitudinally shaped in the direction of said elastic strap (2).

6. Transmitter according to one of the foregoing claims,
**characterized in**
**that** the holes (4, 6) are approximately uniformly distributed over the inner side (7) of said elastic strap (2).

## Revendications

1. Emetteur destiné à la détection du pouls humains pourvu de rubans flexibles (2) qui se tiennent comme des ailes de l'unité d'émetteur (1) et qui présentent à l'intérieur (7) des surfaces de contact électriques de l'unité d'émetteur qui peuvent être mises en contact avec une personne pendant la phase de fonctionnement et lesquels rubans peuvent notamment être tendus au moyen d'un ruban de tension autour d'une cage thoracique, lesdits rubans flexibles (2) comportent des trous (4 à 6 ) dont les sections transversaux libres s'agrandissent de façon progressive dans un état détendu et non dilaté des rubans élastiques à partir des surfaces de contact (7) vers le côté extérieur des rubans flexibles.

2. Emetteur selon la revendication 1, **caractérisé en ce que** les sections transversaux libres des trous ( 4 à 6 ) s'agrandissent vers l'extérieur de manière à peu près exponentielle du côté intérieur (7) des rubans (2) lorsqu'ils sont dans un état détendu et non dilaté.

3. Emetteur selon la revendication 1, **caractérisé en ce que** les sections transversaux libres des trous dans les rubans (2) s'agrandissent vers l'extérieur, lorsqu'ils sont dans un état détendu et non dilaté, en prenant une forme proche d'un cône.

4. Emetteur selon une des revendications 1 à 3, **caractérisé en ce que** les trous (4 à 6 ) sont arrondis sur le côté extérieur des rubans lorsque ceux-ci sont dans un état détendu et non dilaté.

5. Emetteur selon une des revendications 1, 2 et 4, **caractérisé en ce que** les trous ( 4 à 6 ) ont une forme oblongue en direction du ruban flexible lorsqu'il est dans un état détendu et non dilaté.

6. Emetteur selon les revendications précédentes, **caractérisé en ce que** les trous ( 4 à 6 ) sont répartis presque régulièrement au-dessus du côté intérieur (7) du ruban flexible (2) .
